# EUROPEAN PATENT APPLICATION

(11) **EP 2 060 554 A1**
(43) Date of publication of application: **20.05.2009**
(21) Application number: 07398017.9
(22) Date of filing: 19.11.2007
(51) Int. Cl.: C07C 29/84, C07C 31/18, B27K 7/00, B01D 11/02

(54) **Extraction of polyol fractions from cork and cork-derived materials**

(71) Applicant: Amorim Revestimentos, S.A., 4536-907 S. Paio de Oleiros (PT)
(72) Inventor: Pires, Ricardo Alexandre Rodrigues, 1500-548 Lisboa (PT); Chagas, Eng.° José António Marchão das, 4500-669 Silvalde (PT); Reis, Rui Luís Gonçalves dos, 4250-242 Porto (PT)
(74) Representative: Alves Moreira, Pedro

(57) **Abstract**

Cork and cork-derived materials (e.g. black condensate) can be used as raw materials in the extraction of valuable chemicals. The composition of these raw materials includes polyphenols and aliphatic alcohols. These structures (and particularly the aliphatic based) have potential to be used as the hydroxyl component in polymer synthesis (e.g. polyurethane or polyester) and is usually referred to as Polyol. This invention reports a straightforward Polyol solvent extraction procedure from cork and cork-derived materials (e.g. black condensate), composed of one to three stages depending on the target application. The final Polyol fraction can present a black or brown colour, having hydroxyl values between 307 to 131mg of KOH/g sample and an overall extraction yield ranging from 37 to 11%.

## Description

### FIELD OF THE INVENTION

Cork and cork-derived materials (e.g. black condensate) are composed of chemicals of particular interest in different fields. Black condensate, a resinous material leached from the cork particles during the preparation of the insulation corkboards, is a complex matrix of different chemicals. This material, usually, have no relevant economical value. On the other hand, Polyols from vegetable sources are of increasing interest when compared to the petroleum-derived Polyols. A significant percentage of cork and cork-derived materials (e.g. black condensate) are composed by a series of multifunctional aliphatic and aromatic alcoholic components (Polyols) that have potential to be used in polymer synthesis (e.g. polyurethanes or polyesters). To use these components in this field an extraction and separation of the target compounds should be done. This invention reports different procedures to isolate Polyol fractions from cork and cork-derived materials (e.g. black condensate) using standard solvent extraction procedures.

### SUMMARY OF THE INVENTION

This invention reports an extraction and separation procedure of Polyol fraction from the cork and cork-derived materials (e.g. black condensate) by means of solvent extraction techniques. The procedure is composed of two main stages, an initial step using an alcoholic solvent will extract, predominantly, the multifunctional alcoholic chemicals, generating an Hydroxyl Rich Extract; a second stage of the procedure is composed by the extraction of the Hydroxyl Rich Extract with an apolar solvent to obtain, predominantly, the alcoholic components of lower polarity (e.g. long chain aliphatic structures). With this procedure, the final material is composed of aliphatic structures with multi-hydroxyl functionalities.

The use of an alkaline alcoholic treatment during the first stage of the extraction procedure increases the hydroxyl value by cleavage of the ester functionalities present in the raw materials, yielding additional hydroxyl groups.

Overall, different modifications of the extraction procedure will generate Polyol fractions with hydroxyl values between 307 and 131mg KOH/g sample and with yields between 37% and 11% of the initial raw materials.

### BACKGROUND OF THE INVENTION

The extraction and isolation of compounds from cork and cork-derived materials (e.g. black condensate) are usually focused on its terpenoid fraction. Procedures of separating Friedelin from black condensate have been reported and patented using solvent extraction techniques (Júnior et al., PT101683A, 1995; Júnior et al., EP 1103539A1, 1999). Additionally, some studies have reported the suitability of the Polyol fraction present in cork for polymer synthesis, as for example polyurethanes (e.g. Cordeiro et al., Ind. Crop. Prod., 6 (1997) 163-167 and Cordeiro et al., Ind. Crop. Prod., 10 (1999) 1-10). The Polyol fractions through their reaction with carboxylic acids can also be used in the synthesis of polyesters.

The extraction of Polyol from cork and cork-derived materials has been centred in time and solvent taking and complex extraction procedures, involving a series of treatments with different organic solvents (e.g. Cordeiro et al., Holzforschung, 56 (2002), 135-142). These methodologies are usually inappropriate for industrialization. Additionally, there is no report on the extraction of Polyol fraction from black condensate, the main by-product of the insulation corkboard industry, and that has no relevant use or economical value.

Taking into consideration the previously described, it would be interesting to develop a straightforward procedure to extract a Polyol fraction from cork and cork-related materials using a simple one or two step solvent extraction procedure based on usual industrial solvents.

The aim of the present invention is to address this subject and to describe a simple straightforward procedure to extract a Polyol fraction from cork and cork-derived materials, exploring new possible application for the chemical components present in cork.

### DETAILED DESCRIPTION OF THE INVENTION

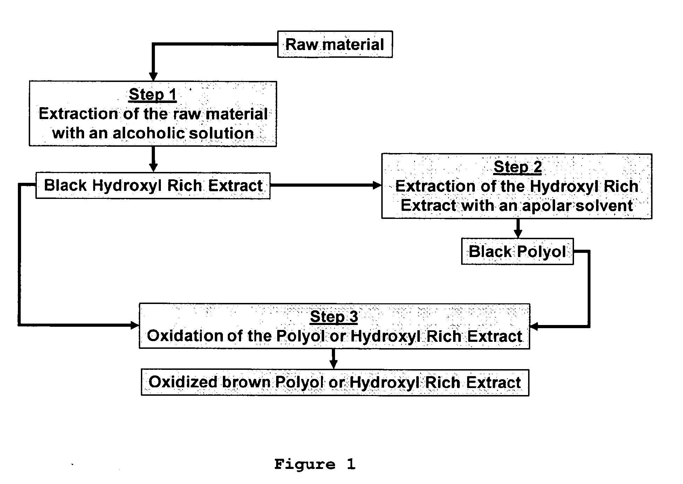

The present invention is based on one or two steps solvent extraction procedure (figure 1) to obtain a Polyol fraction from cork and cork-derived materials (e.g. black condensate). An initial extraction of a Hydroxyl Rich Extract is made using an aliphatic alcoholic solution at different time periods and temperatures. The use of an alkaline (e.g. sodium hydroxide) solution of the alcohol (e.g. ethanol) will generate extracts with increased hydroxyl values due to the induced cleavage of the ester functionalities.

The solution of the extract is separated from the raw material by means of filtration and the extract is neutralized with a concentrated acid (e.g. hydrochloric acid). The neutralization residue is filtered and the extract is obtained after evaporation of the solvent until dryness (that may be recycled).

Depending on the extract obtained from the first stage, it might be necessary a second extraction stage. In this case the Hydroxyl Rich Extract, obtained from the previous step, is extracted with an apolar solvent (e.g. methylene chloride). After filtration of the residue a black Polyol extract is obtained by evaporation of the solvent until dryness (that may also be reused in the process).

A final optional step that may be considered (depending on the final application) is the use of an oxidizing agent (e.g. hydrogen peroxide) with the Hydroxyl Rich Extract from the first stage or the black Polyol extracted from the second stage to generate a brown Polyol or Hydroxyl Rich fraction.

The present examples are based on black condensate due to its complex matrix, although, the described procedures can also be applied to different cork and cork-derived materials.

### EXAMPLE 1

Black condensate (m=151.09g) was ground and solubilized in 1.5L of ethanol, at room temperature and with constant stirring. The residue was separated by filtration and the solution evaporated until dryness, yielding 43.07g of a Hydroxyl Rich Extract.

This example generated an extract with a hydroxyl value of 140mg of KOH/g sample (determined according to the standard ASTM D4274-94) and an acid value of 36mg of KOH/g sample. The overall yield of extraction was 28%.

### EXAMPLE 2

Black condensate (m=151.12g) was ground and solubilized in 1.5L of ethanol, at room temperature and constant stirring. The residue was separated by filtration and the solution was evaporated until dryness, yielding 42.90g of a Hydroxyl Rich Extract.

The Hydroxyl Rich Extract (m=42.90g) was solubilized in 430mL of methylene chloride for 30 minutes at room temperature under constant magnetic stirring. The residue was separated by filtration and the solution was evaporated until dryness, yielding 31.49g of black Polyol fraction.

This example generated a black Polyol fraction with a hydroxyl value of 136mg of KOH/g sample (determined according to the standard ASTM D4274-94) and an acid value of 37mg of KOH/g sample. The overall yield of extraction was 21%.

### EXAMPLE 3

Black condensate (m=215.76g) was ground and extracted with 2% sodium hydroxide ethanolic solution, during 6 hours, under reflux and constant mechanical stirring. The residue was separated by filtration. The solution was neutralized using hydrochloric acid and the neutralization residue separated by filtration. The extraction solution was evaporated until dryness yielding 79.59g of Hydroxyl Rich Extract.

This example generated a black Hydroxyl Rich Extract with a hydroxyl value of 307mg of KOH/g sample (determined according to the standard ASTM D4274-94) and an acid value of 8mg of KOH/g sample. The overall yield of extraction was 37%.

### EXAMPLE 4

Black condensate (m=207.66g) was ground and extracted with 2% sodium hydroxide ethanolic solution, during 6 hours, under reflux and constant mechanical stirring. The residue was separated by filtration. The solution was neutralized using hydrochloric acid and the neutralization residue separated by filtration. The extraction solution was evaporated until dryness yielding 67.43g of Hydroxyl Rich Extract.

The Hydroxyl Rich Extract (m=67.43g) was solubilized in 680mL of methylene chloride for 30 minutes at room temperature under constant magnetic stirring. The residue was separated by filtration and the solution was evaporated until dryness, yielding 33.26g of black Polyol fraction.

This example generated a black Polyol fraction with a hydroxyl value of 145mg of KOH/g sample (determined according to the standard ASTM D4274-94) and an acid value of 6mg of KOH/g sample. The overall yield of extraction was 16%.

### EXAMPLE 5

Black condensate (m=213.81g) was ground and extracted with 2% sodium hydroxide ethanolic solution, during 6 hours, under reflux and constant mechanical stirring. The residue was separated by filtration. The solution was neutralized using hydrochloric acid and the neutralization residue separated by filtration. The extraction solution was evaporated until dryness yielding 74.18g of Hydroxyl Rich Extract.

The Hydroxyl Rich Extract (m=74.18g) was solubilized in 750mL of methylene chloride for 30 minutes at room temperature under constant magnetic stirring. The residue was separated by filtration and the solution was evaporated until dryness, yielding 37.63g of black Polyol fraction.

The black Polyol fraction (m=37.63g) was oxidized using 140mL of hydrogen peroxide during 30 min and under constant magnetic stirring, generating 24.53g of a brown Polyol fraction that was collected by solvent evaporation.

This example generated a brown Polyol fraction with a hydroxyl value of 177mg of KOH/g sample (determined according to the standard ASTM D4274-94) and an acid value of 30mg of KOH/g sample. The overall yield of extraction was 11%.

### EXAMPLE 6

Black condensate (m=219.51g) was ground and extracted with 3% potassium hydroxide methanolic solution, during 3 hours, under reflux and constant mechanical stirring. The residue was separated by filtration. The solution was neutralized using hydrochloric acid and the neutralization residue separated by filtration. The extraction solution was evaporated until dryness yielding 39.15g of Hydroxyl Rich Extract.

The Hydroxyl Rich Extract (m=39.15g) was solubilized in 400mL of methylene chloride for 30 minutes at room temperature under constant magnetic stirring. The residue was separated by filtration and the solution was evaporated until dryness, yielding 26.05g of black Polyol fraction.

This example generated a black Polyol fraction with a hydroxyl value of 131mg of KOH/g sample (determined according to the standard ASTM D4274-94) and an acid value of 21mg of KOH/g sample. The overall yield of extraction was 12%.

## Claims

1. Extraction procedure by solvents using cork or cork-derived materials (e.g. black condensate) as raw materials **characterized by** one or two extraction steps using as solvents an alcohol or alcoholic solution and/or an apolar solvent, in order to obtain a black Hydroxyl Rich Extract or a Polyol fraction, which can be oxidized to yield a brown Hydroxyl Rich Extract or Polyol.

2. Extraction procedure according to claim 1 **characterized by** the first step which is composed of an extraction using an aliphatic alcohol or aliphatic alcohol solution between room temperature to the boiling temperature of the solvent, and during time periods up to 24 hours to obtain a black Hydroxyl Rich Extract.

3. Extraction procedure according to claim 2 **characterized by** the preferred aliphatic alcohols being methanol or ethanol.

4. Extraction procedure according to claims 2 and 3 **characterized by** the use of an alkaline aliphatic alcohol solution that produces an extract with increased hydroxyl value.

5. Extraction procedure according to claim 4 **characterized by** the preferred alkalis being sodium or potassium hydroxide.

6. Extraction procedure **characterized by** the extraction solution obtained by the procedure described in claim 2, 3, 4 and 5 being separated from the residue of extracted raw material by filtration.

7. Extraction procedure according to claim 6 **characterized by** the referred solution should be neutralized using an acid.

8. Extraction procedure according to claim 7 **characterized by** the preferred acid is hydrochloric acid.

9. Extraction procedure according to claim 7 **characterized by** the neutralization precipitate obtained can be collected by filtration;

10. Extraction procedure **characterized by** the evaporation of the solvent of the solution obtained according to claims 6 and 9, until dryness, will produce a black Hydroxyl Rich Extract;

11. Extraction procedure of claims 1 to 10 **characterized by** a second extraction step on the black Hydroxyl Rich Extract using an apolar solvent (e.g. methylene chloride) producing a dissolved extract of predominantly aliphatic structures with hydroxyl functionalities;

12. Extraction procedure of claim 11 **characterized by** the separation of the undissolved residue from the extract is done by filtration;

13. Extraction procedure of claim 12 **characterized by** the evaporation of the solvent until dryness, which will produce a black Polyol fraction that can be used for polymer synthesis;

14. Procedure according to claims 10 or 13 **characterized by** a third step of oxidation using an oxidizing agent (e.g. hydrogen peroxide) that will produce a brown Hydroxyl Rich Extract or Polyol fractions, that can be used as raw material in polymer synthesis.

## Amended claims

### Amended claims in accordance with Rule 137(2) EPC.

**1.** A Process for polyol extraction from cork or cork-derived materials **characterized by** comprising the following steps of:
(i) solvent extraction of said materials with an aliphatic alcohol or a solution thereof, between room temperature to the boiling temperature of the solvent, for up to 24 hours;
(ii) separation of extracted solution by filtration and neutralization with a concentrated acid;
(iii) separation of neutralized residue by filtration and evaporation of solvent until dryness, thus obtaining a so-called black Hydroxyl Rich Extract which can be further extracted;
optionally followed by:
(iv) solvent extraction of residue with an apolar solvent; and
(v) separation of extracted solution by filtration and evaporation of solvent until dryness, thus obtaining a so-called black Polyol Fraction;

**2.** A Process for polyol extraction according to claim 1 **characterized in that** the cork-derived material is black condensate.

**3.** A Process for polyol extraction according to claim 1 and 2 **characterized in that** the aliphatic alcohol is methanol or ethanol.

**4.** A Process for polyol extraction according to claims 1 to 3 **characterized in that** the aliphatic alcohol solution further comprises an alkali.

**5.** A Process for polyol extraction according to claim 4 **characterized in that** the alkali is sodium hydroxide or potassium hydroxide.

**6.** A Process for polyol extraction according to previous claims **characterized in that** the concentrated acid is hydrochloric acid.

**7.** A Process for polyol extraction according to previous claims **characterized in that** the apolar solvent of step (iv) is methylene chloride.

**8.** A process for polyol extraction according to previous claims **characterized by** comprising a further step of oxidation using an oxidizing agent, thus obtaining so-called light brown Hydroxyl Rich Extract or Polyol fractions.

**9.** A Process for polyol extraction according to claim 8 **characterized in that** the oxidizing agent is hydrogen peroxide.

**10.** Use of black Polyol Fraction obtained by the process according to previous claims as a component in polymer synthesis.

**11.** Use of light brown Hydroxyl Rich Extract or Polyol fractions obtained by the process according to claims 8 and 9 as a raw material in polymer synthesis.

**12.** Use according to claim 10 and 11 wherein said polymer synthesis is the synthesis of polyurethanes or polyesters.
